# EUROPEAN PATENT APPLICATION

(11) **EP 4 674 381 A1**
(43) Date of publication of application: **07.01.2026**
(21) Application number: 24777573.7
(22) Date of filing: 22.02.2024
(51) Int. Cl.: A61F 2/24, A61B 17/00

(54) **INTERVENTIONAL HANDLE AND INTERVENTIONAL SYSTEM**

(30) Priority: 31.03.2023 CN 202310333351
(71) Applicant: SHANGHAI MICROPORT CARDIOFLOW MEDTECH CO., LTD., Shanghai 201203 (CN)
(72) Inventor: LU, Shigui, Shanghai 201203 (CN); LIN, Xing, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2024/078023
(87) International publication number: WO 2024/198782

(57) **Abstract**

An interventional handle and an interventional system are disclosed. The interventional handle includes a screw shaft (10), a ball nut (20) and a drive assembly (30). The screw shaft (10) defines a through bore (12), which extends therethrough the screw shaft (10) along its axis and is adapted for coaxial insertion of a catheter assembly (40) therein. The screw shaft (10) is adapted for coaxial connection with the catheter assembly (40). The ball nut (20) is in engagement with the screw shaft (10) so as to be rotatable circumferentially around the screw shaft (10) and is restricted from displacement along the axis of the screw shaft (10). The drive assembly (30) is adapted to drive the ball nut (20) to circumferentially rotate around the screw shaft (10), causing axial translation of the screw shaft (10). With this arrangement, since the catheter assembly (40) is coaxially inserted in the through bore (12) of the screw shaft (10) and coaxially coupled to the screw shaft (10), it exerts only axial forces on the screw shaft (10), effectively overcoming the issues associated with a conventional eccentric catheter (03).

## Description

### TECHNICAL FIELD

The present invention relates to the field of medical devices, and more particularly to a handle and system for interventional use.

### BACKGROUND

Interventional therapy is a brand new therapeutic technique, which has emerged in recent years as a minimally invasive alternative therapeutic option based on modern high technologies. It involves introducing, into a patient's body under the guidance of medical imaging equipment, a specialized precision instrument for diagnosis and local treatment of a lesion therein. This technique features minor trauma, fast recovery and good outcomes, and avoids possible harm of traditional surgery to patients.

Delivery systems used in interventional procedures are generally composed of catheters and a handle. Serving as a power source for the entire procedure, the handle is required to provide sufficient safety and effectiveness. To date, conventional manual handles rely on threaded fits for power transmission, while electric handles employ a motor, a shaft coupling, gears, a screw and other components for power transmission. Some applications require the use of a delivery system with higher accuracy, greater ease of operation and other improved aspects of performance, such as more efficient power transmission, increased coaxiality of components involved in power transmission to a catheter, and steerability of a catheter.

Some conventional handles use a screw and a nut as a conversion mechanism for power transmission, with a catheter being arranged eccentrically on one side of the screw. This arrangement not only occupies a large space, but also creates a deflection torque exerted by the eccentric catheter on the screw-nut mechanism, which may lead to the screw-nut power transmission getting stuck, or even failure or damage thereto.

### SUMMARY

It is an object of the present invention to provide a handle and system for interventional use, which overcome the problems associated with conventional handles arising from the use of an eccentric catheter.

To this end, the present invention provides an interventional handle, which includes a screw shaft, a ball nut and a drive assembly,
the screw shaft defining a through bore, which extends through the screw shaft along its own axis and is adapted for coaxial insertion of a catheter assembly therein, the screw shaft adapted for coaxial connection with the catheter assembly,
the ball nut in engagement with the screw shaft so as to be rotatable circumferentially around the screw shaft and restricted from displacement along the axis of the screw shaft,
the drive assembly adapted to drive the ball nut to circumferentially rotate around the screw shaft, thereby causing axial translation of the screw shaft

Optionally, the screw shaft may have a connecting portion adapted for coaxial connection with the catheter assembly along the axis of the screw shaft at a joint rotationally symmetrical about the axis of the screw shaft.

Optionally, the connecting portion may be continuous over an entire circumference of an inner wall of the through bore and attached to the catheter assembly over an entire outer circumferential wall thereof.

Optionally, the handle may further include a stop assembly,
the stop assembly switchable between a locking configuration and an unlocking configuration,
the stop assembly, when in the locking configuration, restricting an axial travel length of the screw shaft within a predetermined travel length range,
the stop assembly, when in the unlocking configuration, not restricting the axial travel length of the screw shaft anymore and allowing it to exceed the predetermined travel length range.

Optionally, the stop assembly may include a protrusion member and a locking member, the protrusion member projecting from an outer wall of the screw shaft, the locking member movable between a first position and a second position in a direction perpendicular to the axis of the screw shaft, wherein:
when the locking member is located at the first position, there is an overlap between projections of the locking member and the protrusion member along the axis of the screw shaft, and the stop assembly is in the locking configuration, in which when the axial travel length of the screw shaft reaches a limit of the predetermined travel length range, the protrusion member comes into abutment with the locking member; and
when the locking member is located at the second position, there is no overlap between projections of the locking member and the protrusion member along the axis of the screw shaft, and the stop assembly is in the unlocking configuration.

Optionally, the stop assembly may further include a biasing member adapted to bias the locking member from the first position toward the second position so that the locking member remains at the second position when not stressed or restricted.

Optionally, the stop assembly may further include a detachable or movable manipulation member for urging the locking member from the second position into the first position.

Optionally, the manipulation member may be disposed so able to be movable between a third position and a fourth position along the axis of the screw shaft and may include a push-pull section, a sloped section and an abutting section, which are joined in sequence along the axis of the screw shaft, wherein:
when the manipulation member is at the third position, the push-pull section is axially aligned with the locking member, and the locking member is at the second position;
while the manipulation member is being moved from the third position toward the fourth position, the sloped section gradually urges the locking member from the second position toward the first position; and
when the manipulation member is at the fourth position, the abutting section is in axial alignment and abutment with the locking member, thereby retaining the locking member at the first position.

Optionally, the handle may further include a housing and the manipulation member may include a detachable cover, wherein:
when the cover is assembled on the housing, it abuts against the locking member, thereby retaining the locking member at the first position; and
when the cover is detached and separated from the housing, the locking member is at the second position.

To the above end, the present invention also provides a system for interventional use, which includes the handle as defined above and a catheter assembly inserted within the through bore and coupled to the screw shaft.

As noted above, the present invention provides a handle and system for interventional use. The handle includes a screw shaft, a ball nut and a drive assembly. The screw shaft defines a through bore, which extends therethrough the screw shaft along its axis and is adapted for coaxial insertion of a catheter assembly therein. The screw shaft is adapted for coaxial connection with the catheter assembly. The ball nut is in engagement with the screw shaft so as to be rotatable circumferentially around the screw shaft and is restricted from displacement along the axis of the screw shaft. The drive assembly is adapted to drive the ball nut to circumferentially rotate around the screw shaft, causing axial translation of the screw shaft.

With this arrangement, since the catheter assembly is coaxially inserted in the through bore of the screw shaft and coaxially coupled to the screw shaft, it exerts only axial forces on the screw shaft, effectively overcoming the issues associated with a conventional eccentric catheter.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a schematic axial cross-sectional view of an interventional handle in relation to the present invention;
Fig. 2 shows a schematic axial cross-sectional view of an interventional handle according to an embodiment of the present invention;
Fig. 3 schematically illustrates the handle of Fig. 2, in which a screw shaft has undergone proximal translation;
Fig. 4 shows a schematic partial view of an interventional handle according to an embodiment of the present invention, which includes a power transmission member implemented as a gear set;
Fig. 5 shows a schematic partial view of an interventional handle according to an embodiment of the present invention, which includes a power transmission member implemented as a belt transmission mechanism;
Fig. 6 shows a schematic partial view of an interventional handle according to an embodiment of the present invention, which includes a motor implemented as a coreless motor;
Fig. 7 schematically illustrates a screw shaft in engagement with a ball nut according to an embodiment of the present invention;
Fig. 8 shows a schematic overview of an interventional handle according to an embodiment of the present invention;
Fig. 9 shows a schematic partial view of an interventional handle according to an embodiment of the present invention, which includes a stop assembly;
Fig. 10 shows a schematic axial cross-sectional view of an interventional handle according to an embodiment of the present invention, which includes a stop assembly;
Fig. 11 is a schematic diagram showing a stop assembly in a locking configuration and a screw shaft located within a predetermined travel length range according to an embodiment of the present invention;
Fig. 12 is a schematic diagram showing a stop assembly in a locking configuration and a screw shaft located at a limit of a predetermined travel length range according to an embodiment of the present invention;
Fig. 13 is a schematic diagram showing a stop assembly in an unlocking configuration according to an embodiment of the present invention;
Fig. 14 is a schematic diagram showing a cover assembled on a housing according to an embodiment of the present invention; and
Fig. 15 is a schematic diagram showing a cover separated from a housing according to an embodiment of the present invention.

### List of Reference Numerals

01 screw; 02 nut; 03 catheter;
10 screw shaft; 11 connecting portion; 12 through bore; 20 ball nut; 30 drive assembly; 31 manual actuator; 311 rotary knob; 32 electric actuator; 321 motor; 322 power transmission member; 40 catheter assembly; 50 housing; 60 stop assembly; 61 protrusion member; 62 locking member; 64 manipulation member; 641 push-pull section; 642 sloped section; 643 abutting section; 644 cover.

### DETAILED DESCRIPTION

Objects, advantages and features of the present invention will become more apparent upon reading the following more detailed description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" of "at least one" and "at least two" of "two or more". Additionally, the use of the terms "first", "second" and "third" herein is intended for illustration only and is not to be construed as denoting or implying relative importance or as implicitly indicating the numerical number of the referenced items. Accordingly, defining an item with "first", "second" or "third" is an explicit or implicit indication of the presence of one or at least two such items. The terms "one end" and "the other end", as well as "proximal end" and "distal end", may be used herein to generally refer to corresponding end portions, rather than precisely to the endpoints. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

It is an object of the present invention to provide a handle and system for interventional use, which overcome the problems associated with conventional handles arising from the eccentricity of a catheter, as described below with reference to the accompany drawings.

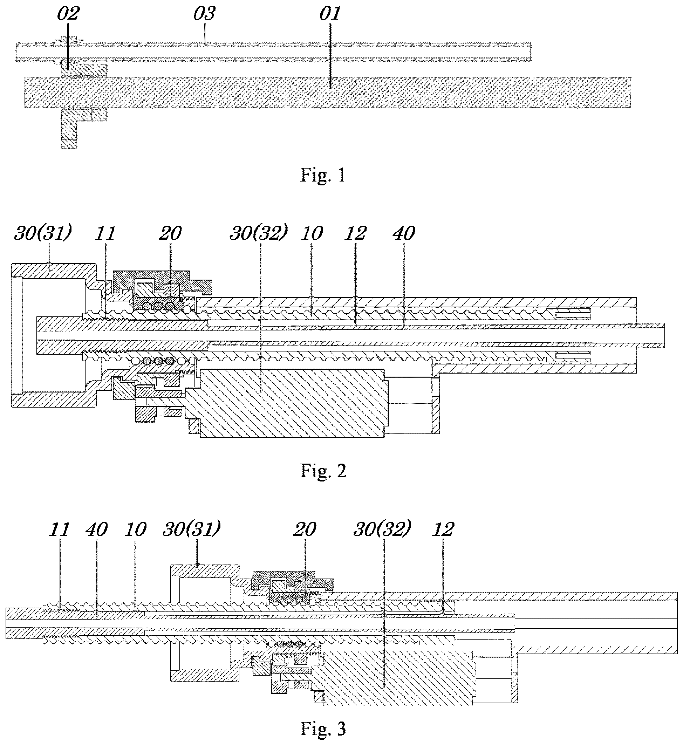

Referring to Fig. 1, an interventional handle includes a screw 01, a nut 02 and a catheter 03. The screw 01 can be rotated about its own axis, the position of the screw 01 along its own axis is limited. The nut 02 is in threaded engagement with the screw 01 and the movement of the screw 01 is limited to circumferential rotation around the screw 01. The catheter 03 is attached to one side of the nut 02, allowing rotation of the screw 01 about its own axis causes translation of the nut 02 along the axis of the screw 01 and hence the catheter 03 along the axis. As described in the Background section, with this arrangement, the screw 01 and the nut 02 occupy a large space, which is detrimental to miniaturization of the handle. Additionally, when encountering resistance, the catheter 03 may apply a deflection torque to the screw 01, which may bend or deflect, to a certain extent, the screw 01 that is supposed to be subject only to forces along its axis. Such deflection torques are very unfavorable for elongate components such as the screw 01 because a large deflection torque tends to cause damage to the screw 01. As a consequence, the nut 02 may get stuck on the screw 01, leading to failure in power transmission.

In view of the above, as shown in Figs. 2 and 3, embodiments of the present invention provide an interventional handle, which includes a screw shaft 10, a ball nut 20 and a drive assembly 30. The screw shaft 10 defining a through bore 12 extending through the screw shaft along its axis, which is adapted for coaxial insertion of a catheter assembly 40 therein. The screw shaft 10 is adapted for coaxial connection with the catheter assembly 40. The ball nut 20 is in engagement with the screw shaft 10 so as to be circumferentially rotatable around the screw shaft 10 and is restricted from displacement along the axis of the screw shaft 10. The drive assembly 30 is adapted to drive the ball nut 20 to circumferentially rotate around the screw shaft 10, causing axial translation of the screw shaft 10. Since the catheter assembly 40 is coupled to the screw shaft 10, the axial translation of the screw shaft 10 will cause translation of the catheter assembly 40 or part thereof in the same direction.

Compared with the handle of Fig. 1, in this embodiment, the catheter assembly 40 is not coupled to the ball nut 20, but to the screw shaft 10. Moreover, the catheter assembly 40 is directly inserted (partially) in the through bore 12 of the screw shaft 10. Further, the ball nut 20 is restricted in axial position and the screw shaft 10 is restricted from circumferential rotation, enabling the screw shaft 10 to axially translate as a result of rotation of the ball nut 20, thereby coaxially actuating the catheter assembly 40 to move in the same way. With this arrangement, the catheter assembly 40 exerts only axial forces on the screw shaft 10, effectively overcoming the issues associated with a conventional eccentric catheter.

As shown in Fig. 3, in one exemplary embodiment, the drive assembly 30 includes a manual actuator 31, the manual actuator 31 includes a rotary knob 311, the rotary knob 311 is coupled, preferably coaxially and fixedly, to the ball nut 20. In this way, rotating the rotary knob 311 will cause rotation of the ball nut 20. Preferably, the rotary knob 311 defining a through bore extending therethrough along the axis of the screw shaft 10, and the screw shaft 10 can be inserted through the bore, as shown in Fig. 3.

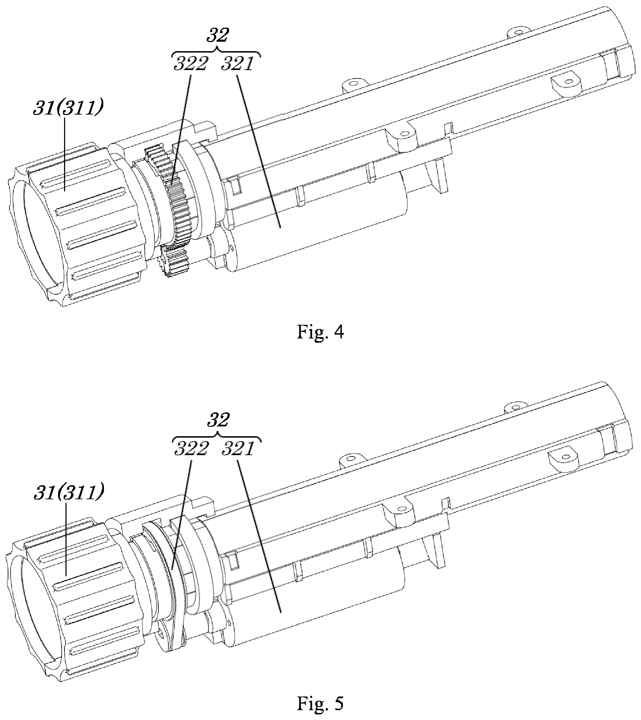

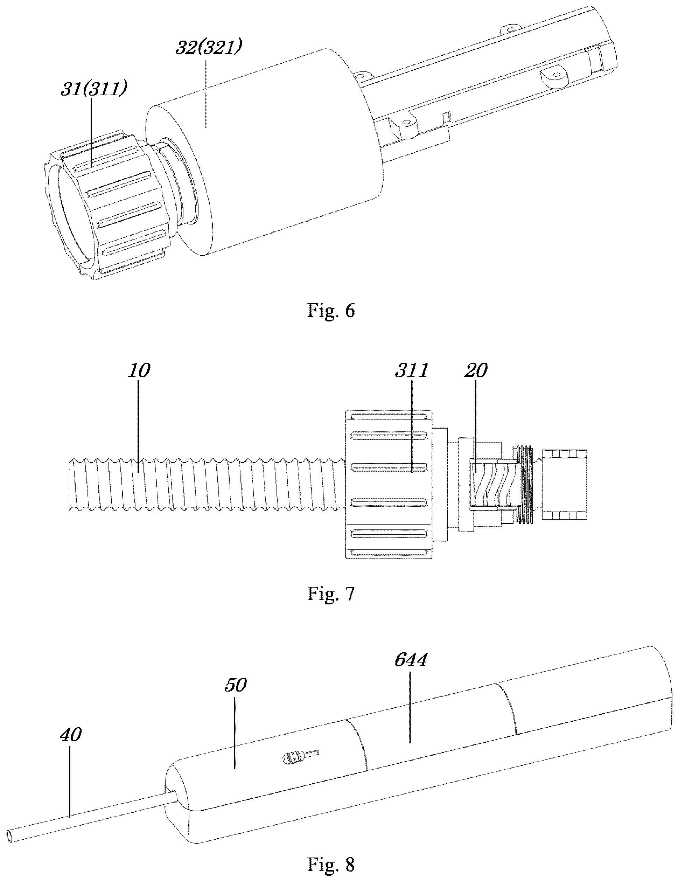

As shown in Figs. 4 and 5, in another exemplary embodiment, the drive assembly 30 includes an electric actuator 32, the electric actuator 32 includes a motor 321 and a power transmission member 322. The motor 321 is connected to the ball nut 20 via the power transmission member 322. The power transmission member 322 includes, for example, a gear set (as shown in Fig. 4), or a belt transmission mechanism (as shown in Fig. 5). As shown in Fig. 6, in some alternative embodiments, the power transmission member 322 may be omitted from the electric actuator 32. In this case, for example, the motor 321 may be implemented as a coreless motor and used to directly actuate the ball nut 20. Those skilled in the art will understand how the electric actuator 32 is structured and operates based on the knowledge in the art.

It should be noted that the drive assembly 30 may include both the manual actuator 31 and the electric actuator 32. In a manual actuation mode, the rotary knob 311 may be directly rotated to cause the electric actuator 32 to rotate in the same way, without encountering any resistance. In an electric actuation mode, the motor 321 may output power to drive the ball nut 20 and hence the rotary knob 311 to rotate.

As shown in Fig. 7, in the present embodiment, power may be transmitted between the screw shaft 10 and the ball nut 20 through balls. This can provide power transmission efficiency as high as 90%, or higher, with less power loss. Thus, for the same loading, the motor 321 is allowed to be selected as a smaller, more inexpensive motor with a lower torque output. Optionally, the ball nut 20 may employ either an internal or external ball return mechanism, without departing from the scope of the present invention. The ball nut 20 may be structured as known in the art, and further description thereof is omitted herein.

Preferably, in the present embodiment, the screw shaft 10 is made of a polymeric material, which allows for easy fabrication and easy formation of the through bore 12. More specifically, since the screw shaft 10 will not be subject to any deflection torque during use, fabricating the screw shaft 10 with a polymeric material can ensure sufficient tensile and compressive strength, dispensing the use of a metallic material. This can significantly reduce the cost and the fabrication complexity, while ensuring strength and reliability.

Preferably, the screw shaft 10 has a connecting portion 11, the connecting portion 11 is adapted for coaxial connection with the catheter assembly 40 along the axis of the screw shaft 10. Optionally, the resultant of forces transmitted between the connecting portion 11 and the catheter assembly 40 may pass along the axis of the screw shaft 10. In one example, the joint of the connecting portion 11 and the catheter assembly 40 is rotationally symmetrical about the axis of the screw shaft 10. It should be noted that the "joint" of the connecting portion 11 and the catheter assembly 40 refers to a portion of the connecting portion 11, where it is joined to the catheter assembly 40 and subject to forces exerted thereby. Moreover, the term "rotationally symmetrical" conventionally refers to the coincidence of a shape in plane that has been rotated about a fixed point in the plane by an angle of α (0°<α<360°) with the original shape. Such a shape is called a rotationally symmetrical shape. However, as used herein, the term is intended to be more broadly construed to mean that after the joint of the connecting portion 11 and the catheter assembly 40 is rotated by an angle of α (0°<α<360°) about the axis of the screw shaft 10, it will come into coincidence with its original shape. For example, the connecting portion 11 may be joined to the catheter assembly 40 at multiple points, which are, for example, evenly spaced apart circumferentially around the axis of the screw shaft 10 (e.g., the multiple points define a regular polygon). As would be appreciated, the rotational symmetry of the joint of the connecting portion 11 and the catheter assembly 40 about the axis of the screw shaft 10 leads to the resultant of forces transferred between the connecting portion 11 and the catheter assembly 40 passing along the axis of the screw shaft 10. As a result, when subject to any resistance or pulling force, the catheter assembly 40 will exert a force on the screw shaft 10 simply along its axis, without creating any deflection torque thereon.

If the joint of the connecting portion 11 and the catheter assembly 40 is not rotationally symmetrical about the axis of the screw shaft 10, the catheter assembly 40 will apply a more or less deflection torque to the screw shaft 10. For example, if the catheter assembly 40 is joined to the connecting portion 11 at a single point despite its coaxial insertion in the through bore 12, the joint will obviously not be rotationally symmetrical about the axis of the screw shaft 10. In this case, when subject to a resistance or pulling force, the catheter assembly 40 may apply a deflection torque to the screw shaft 10, making this design undesirable.

Preferably, the connecting portion 11 is continuous over the entire circumference of an inner wall of the through bore 12 and is attached to the catheter assembly 40 over its entire outer circumferential wall. With this arrangement, force transmission between the connecting portion 11 and catheter assembly 40 will occur in a circumferentially uniform manner, without creating any deflection torque. In an alternative exemplary embodiment, the connecting portion 11 is implemented as an internal thread formed in the inner wall of the through bore 12, and correspondingly, there is a mating external thread on the outer wall of the catheter assembly 40. Once the catheter assembly 40 is screwed into the connecting portion 11, uniform force transmission can be achieved circumferentially. Moreover, the catheter assembly 40 can be easily disengaged from the connecting portion 11. That is, the catheter assembly 40 is detachably coupled to the connecting portion 11. Thus, when there is a need for another catheter assembly 40 of a different size, the catheter assembly 40 can be easily detached and replaced with the other catheter assembly 40. Of course, the present invention is not limited to any particular method for coupling the connecting portion 11 to the catheter assembly 40, and in some other embodiments, the coupling may be accomplished, for example, by snap engagement, adhesive bonding, an interference fit, or the like. Modifications and alternatives are possible to those skilled in the art in this regard.

Fig. 8 shows use of the handle for delivery of a prosthetic valve in an application involving interventional therapy of a heart valve. Optionally, the handle may further include a housing 50, and the catheter assembly 40 may include an inner tube (not shown) and an outer tube. The inner tube is movably within the outer tube and fixedly coupled to the housing 50, and the outer tube is coupled to the connecting portion 11. The prosthetic valve is disposed between the inner and outer tubes. During use, after the inner and outer tubes, along with the prosthetic valve sandwiched therebetween, are advanced to a target site, the housing 50 and hence the inner tube may be held stationary, and the drive assembly 30 may be then manipulated to advance or retract the screw shaft 10 along its axis, causing the outer tube to move in the same way. In this way, the outer tube can be displaced relative to the inner tube, allowing for loading, release or retrieval of the prosthetic valve. In particular, the catheter assembly 40 may be structured and operate as known in the art, and further description thereof is omitted herein.

In an application involving interventional therapy of a heart valve, it may be necessary for the outer tube to be moved axially to allow for loading, release or retrieval of a prosthetic valve. In some specific applications, after the inner and outer tubes are advanced, together with a prosthetic valve being sandwiched therebetween, to a target site, the outer tube may be caused to move proximally (i.e., toward the operator, or away from the lesion) to partially release the prosthetic valve. After that, it may be determined whether the valve has been released at a proper location, for example, based on a radiographic image. If not, the outer tube may be caused to move distally (i.e., away from the operator, or toward the lesion) to retrieve the prosthetic valve, followed by another attempt for partial release at a different location. This may be repeated until the prosthetic valve is partially released at a desired location. Finally, the outer tube may be caused to move proximally to completely release the prosthetic valve.

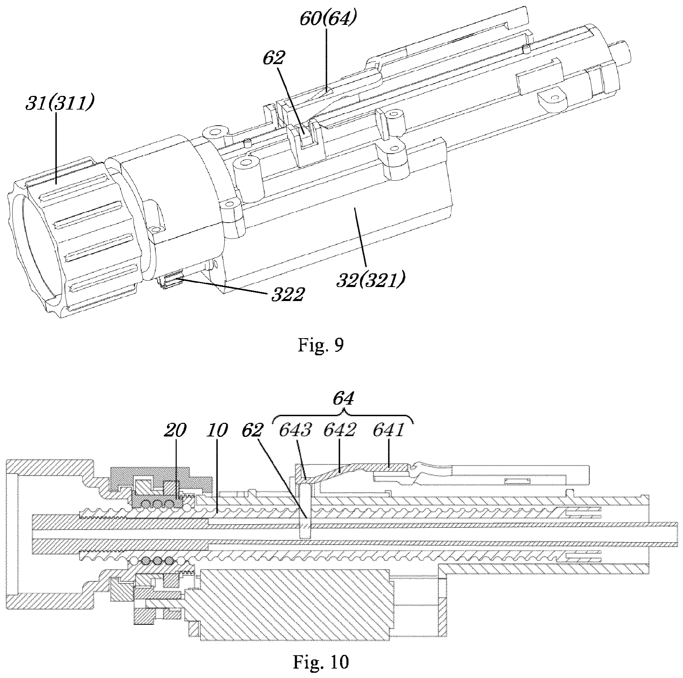

In the above application, during each attempt for partial release of the prosthetic valve, it necessary to ensure that associated proximal movement of the outer tube does not lead to complete release of the prosthetic valve. To this end, referring to Figs. 9 to 15, the handle is preferred to further include a stop assembly 60 switchable between locking and unlocking configurations. When in the stop assembly 60 is in the locking configuration, it restricts a travel length along the axis of the screw shaft 10 within a predetermined travel length range. On the contrary, when the stop assembly 60 is in the unlocking configuration, it does not restrict the travel length along the axis of the screw shaft 10 any longer and allows it to exceed the predetermined travel length range.

It will be understood that the predetermined travel length range may be appropriately determined as required for partial release of the prosthetic valve by manipulating the outer tube. In particular, the predetermined travel length range may depend on, among other factors, an axial length and expandability of the prosthetic valve and initial relative positions of the inner and outer tubes. With this arrangement, when the stop assembly 60 is in the locking configuration, an axial travel length of the screw shaft 10 can be restricted within the predetermined travel length range, ensuring that, in any case, movement of the outer tube that is coupled to the connecting portion 11 will lead to only partial release of the prosthetic valve and that any overly release of the prosthetic valve can be prevented, ensuring that the prosthetic valve can be retrieved. Once the prosthetic valve is partially released at a proper location, the stop assembly 60 may be switched to the unlocking configuration, in which the axial travel length of the screw shaft 10 is no longer restricted, and the outer tube that is coupled to the connecting portion 11 can be proximally moved a length exceeding the predetermined travel length range to allow complete release of the prosthetic valve, as required by its implantation.

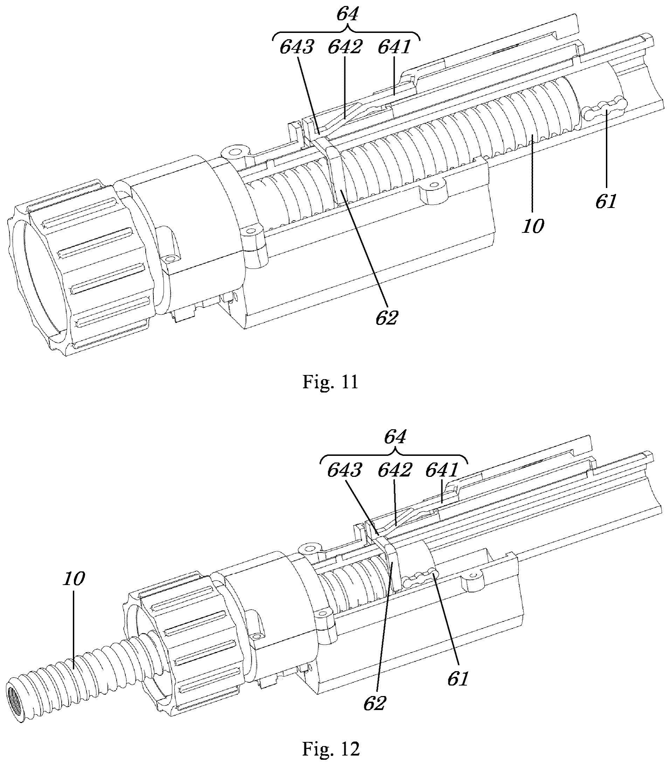

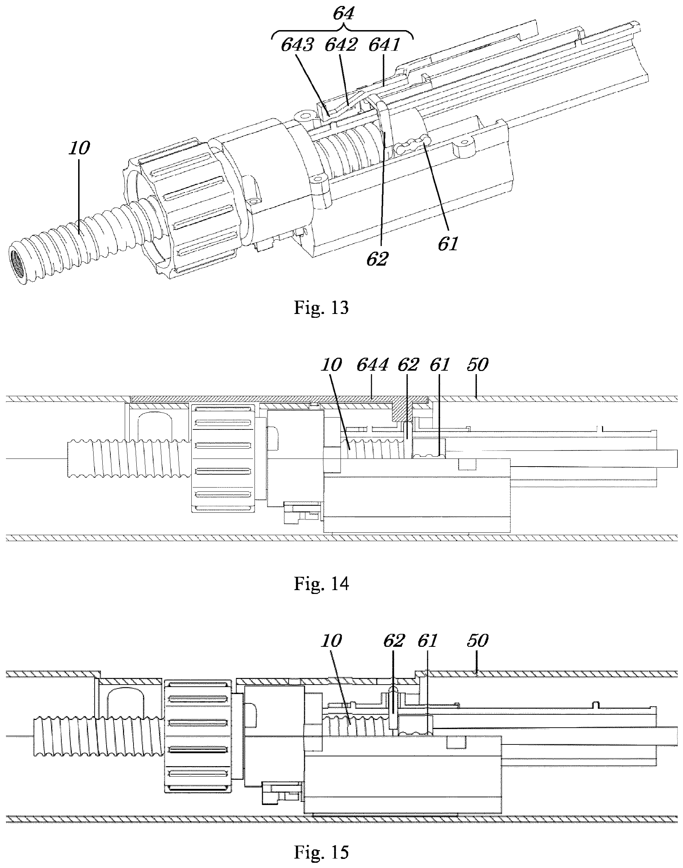

As shown in Figs. 11 to 13, in one exemplary embodiment, the stop assembly 60 includes a protrusion member 61 and a locking member 62. The protrusion member 61 projects from an outer wall of the screw shaft 10, and the locking member 62 is disposed so as to be movable between a first position and a second position in a direction perpendicular to the axis of the screw shaft 10. When the locking member 62 is located at the first position (see Figs. 11 and 12), there is an overlap between projections of the locking member 62 and the protrusion member 61 along the axis of the screw shaft 10, putting the stop assembly 60 into the locking configuration. Accordingly, the protrusion member 61 will come into abutment with the locking member 62 when the travel length along the axis of the screw shaft 10 reaches a limit of the predetermined travel length range (see Fig. 12). When the locking member is located at the second position (see Fig. 13), there is no overlap between projections of the locking member 62 and the protrusion member 61 along the axis of the screw shaft 10, putting the stop assembly 60 into the unlocking configuration. Here, the projections of the locking member 62 and the protrusion member 61 along the axis of the screw shaft 10 refer to projections of their outermost contours in a cross-sectional plane perpendicular to the axis of the screw shaft 10 on another plane perpendicular to the axis of the screw shaft 10.

In the exemplary embodiment of Figs. 11 to 13, the protrusion member 61 projects radially outwards from the screw shaft 10 and is fixed thereto, and the locking member 62 is movable radially with respect to the screw shaft 10 (e.g., vertically, as viewed in the orientation of Figs. 11 to 13). The locking member 62 is an n-shaped component defining an opening slightly greater than an outer diameter of the screw shaft 10, allowing the screw shaft 10 to move therein. When the locking member 62 is lowered to the first position (which is a lower position, as shown in Fig. 12), the protrusion member 61 can come into abutment with it, preventing the screw shaft 10 from further proximal movement (to the left, as viewed in the orientation of Fig. 12). This is equivalent to restricting movement thereof along the axis of the screw shaft 10 within the predetermined travel length range. On the contrary, when the locking member 62 is raised to the second position (which is an upper position, as shown in Fig. 13), the protrusion member 61 will not be restricted anymore, allowing the screw shaft 10 to move more proximally (to the left, as viewed in the orientation of Fig. 12). Preferably, the stop assembly 60 includes two protrusion members 61 arranged in symmetry about the axis of the screw shaft 10, which enable even more stable and even more reliable abutment with the locking member 62.

The stop assembly 60 further includes a biasing member for biasing the locking member 62 from the first position toward the second position. In this way, the locking member 62 remains at the second position when not stressed or restricted. For example, the biasing member may be an elastic member, such as a spring or resilient tab. Alternatively, it may be a magnetic member, for example, consisting of magnets arranged in opposition to create an attractive or repulsive biasing force. The present invention is not limited to any particular structure of the biasing member. With the biasing member, reliable switching of the locking member 62 can be ensured, preventing the locking member 62 from getting stuck at the first position while it is desired to completely release the prosthetic valve.

Optionally, the stop assembly 60 may further include a manipulation member 64, the manipulation member 64 is detachable or movable, the manipulation member 64 is used to apply pressure to the locking member 62 to urge the locking member 62 from the second position into the first position. It will be understood that, under the action of the biasing member, the locking member 62 will rest at the second position by default if the locking member 62 is not stressed or restricted at all. However, in this case, the manipulation member 64 may be manipulated to urge the locking member 62 into the first position by overcoming the action of the biasing member.

As shown in Figs. 9 to 13, in one exemplary embodiment, the manipulation member 64 is disposed so as to be movable between a third position (or proximal position, see Fig. 13) and a fourth position (or distal position, see Fig. 12) along the axis of the screw shaft 10. The manipulation member 64 includes a push-pull section 641, a sloped section 642 and an abutting section 643, which are joined in sequence along the axis of the screw shaft 10. When the manipulation member 64 is located at the third position, the push-pull section 641 is axially aligned with the locking member 62, and the locking member 62 is located at the second position. While the manipulation member 64 is being moved from the third position toward the fourth position, the sloped section 642 will gradually urge the locking member 62 from the second position toward the first position. When the manipulation member 64 is located at the fourth position, the abutting section 643 is in axial alignment with the locking member 62, and the abutting section 643 is in abutment with the locking member 62, retaining the locking member 62 in the first position.

In the exemplary embodiment of Figs. 9 to 13, the sloped section 642 is gradually sloped toward the first position (downwardly, as viewed in the orientation of the figures) in a direction from the fourth position toward the third position (to the left, as viewed in the orientation of the figures). The push-pull section 641 is joined to a distal end of the sloped section 642 (the right-hand end, as viewed in the orientation of the figures), and the abutting section 643 is joined to a proximal end of the sloped section 642 (the lefthand end, as viewed in the orientation of the figures). With this arrangement, while the manipulation member 64 is being moved from the third position toward the fourth position (to the right, as viewed in the orientation of the figures), the sloped section 642 that is sloped toward the first position will gradually urge the locking member 62 toward the first position direction. When the manipulation member 64 reaches the fourth position, the abutting section 643 will completely retain the locking member 62 at the first position.

The exemplary embodiment of Figs. 9 to 13 is presented merely as a non-limiting example of the manipulation member 64. In an alternative exemplary embodiment, as shown in Figs. 14 and 15, the manipulation member 64 includes a detachable cover 644. When the cover 644 is assembled on the housing 50, the cover 644 abuts against the locking member 62, retaining the locking member 62 at the first position. After the cover 644 is detached from the housing 50, the locking member 62 will be located at the second position.

The cover 644 may be assembled on the housing 50, for example, by snap engagement or magnetic attraction. During use, in order to partially release the prosthetic valve, the cover 644 is maintained on the housing 50, preventing overly release of the prosthetic valve. Once it is confirmed that the prosthetic valve is partially released at a desired location, the cover 644 may be separated from the housing 50. As a result, because of not being confined by the cover 644 anymore, the locking member 62 will be biased to the second position, unlocking the screw shaft 10 and thus allowing it to move so that the prosthetic valve is completely released.

On the basis of the handle as discussed above, embodiments of the present invention also provide a system for interventional use, which includes the handle and a catheter assembly 40, the catheter assembly 40 is inserted in the through bore 12 and coupled to the screw shaft 10 (e.g., at the connecting portion 11). The system may also include other components, which may be structured and operate as known in the art and, thereby, need not be described in further detail herein.

To sum up, the present invention provides a handle and system for interventional use. The handle includes a screw shaft, a ball nut and a drive assembly. The screw shaft defines a through bore, which extends therethrough the screw shaft along its axis and is adapted for coaxial insertion of a catheter assembly therein. The screw shaft is adapted for coaxial connection with the catheter assembly. The ball nut is in engagement with the screw shaft so as to be rotatable circumferentially around the screw shaft and is restricted from displacement along the axis of the screw shaft. The drive assembly is adapted to drive the ball nut to circumferentially rotate around the screw shaft, causing axial translation of the screw shaft. With this arrangement, since the catheter assembly is coaxially inserted in the through bore of the screw shaft and coaxially coupled to the screw shaft, it exerts only axial forces on the screw shaft, effectively overcoming the issues associated with a conventional eccentric catheter.

It should be noted that the foregoing embodiments may be combined in any suitable combination. The description presented above is merely that of a few preferred embodiments of the present invention and is not intended to limit the scope thereof in any sense. Any and all changes and modifications made by those of ordinary skill in the art based on the above teachings fall within the scope of the invention.

## Claims

1. An interventional handle, comprising a screw shaft, a ball nut and a drive assembly,
the screw shaft provided with a through bore extending through the screw shaft along an axis of the screw shaft, the through bore adapted for a catheter assembly to coaxially pass therethrough, the screw shaft adapted to be coaxially connected to the catheter assembly,
the ball nut fitting and connected to the screw shaft so as to be rotatable around the screw shaft in a circumferential direction thereof, and the ball nut restricted from displacement along the axis of the screw shaft,
the drive assembly adapted to drive the ball nut to rotate around the screw shaft in the circumferential direction thereof, thereby driving the screw shaft to move in an axial direction thereof.

2. The interventional handle according to claim 1, wherein the screw shaft has a connecting portion adapted to be coaxially connected to the catheter assembly along the axis of the screw shaft, and wherein a joint at which the connecting portion connected to the catheter assembly is rotationally symmetrical about the axis of the screw shaft.

3. The interventional handle according to claim 2, wherein the connecting portion is continuous over an entire circumference of an inner wall of the through bore and is attached to the catheter assembly over an entire outer circumferential wall thereof.

4. The interventional handle according to claim 1, further comprising a stop assembly,
the stop assembly switchable between a locking configuration and an unlocking configuration,
the stop assembly, when in the locking configuration, restricting an axial travel length of the screw shaft within a predetermined travel length range,
the stop assembly, when in the unlocking configuration, not restricting the axial travel length of the screw shaft anymore and allowing the axial travel length of the screw shaft to exceed the predetermined travel length range.

5. The interventional handle according to claim 4, wherein: the stop assembly comprises a protrusion member and a locking member, the protrusion member projecting from an outer wall of the screw shaft, the locking member movable between a first position and a second position in a direction perpendicular to the axis of the screw shaft,
when the locking member is located at the first position, there is an overlap between projections of the locking member and the protrusion member along the axis of the screw shaft, and the stop assembly is in the locking configuration, in which when the axial travel length of the screw shaft reaches a limit of the predetermined travel length range, the protrusion member comes into abutment with the locking member; and
when the locking member is located at the second position, there is no overlap between projections of the locking member and the protrusion member along the axis of the screw shaft, and the stop assembly is in the unlocking configuration.

6. The interventional handle according to claim 5, wherein the stop assembly further comprises a biasing member adapted to bias the locking member from the first position toward the second position so that the locking member remains at the second position when not stressed or restricted.

7. The interventional handle according to claim 5 or 6, wherein: the stop assembly further comprises a manipulation member, the manipulation member is detachable or movable, the manipulation member is used to apply a pressure to the locking member to urge the locking member from the second position to the first position.

8. The interventional handle according to claim 7, wherein: the manipulation member is disposed so able to be movable between a third position and a fourth position along the axis of the screw shaft, and the manipulation member comprises a push-pull section, a sloped section and an abutting section, which are joined in sequence along the axis of the screw shaft,
when the manipulation member is at the third position, the push-pull section is axially aligned with the locking member, and the locking member is at the second position;
while the manipulation member is being moved from the third position toward the fourth position, the sloped section gradually urges the locking member from the second position toward the first position; and
when the manipulation member is at the fourth position, the abutting section is in axial alignment with the locking member, and the abutting section is in abutment with the locking member, thereby retaining the locking member at the first position.

9. The interventional handle according to claim 7, wherein: the interventional handle further comprises a housing, the manipulation member comprising a cover which is detachable,
when the cover is assembled on the housing, the cover abuts against the locking member, thereby retaining the locking member at the first position; and
when the cover is detached and separated from the housing, the locking member is at the second position.

10. A system for interventional use, comprising the interventional handle according to any one of claims 1 to 9 and the catheter assembly inserted within the through bore and coupled to the screw shaft.
